# EUROPÄISCHE PATENTANMELDUNG

(11) **EP 4 748 421 A1**
(43) Veröffentlichungstag der Anmeldung: **27.05.2026**
(21) Anmeldenummer: 25217053.5
(22) Anmeldetag: 19.11.2025
(51) Int. Cl.: A61M 16/00, A61M 16/12, A61M 16/20

(54) **VORRICHTUNG ZUM APPLIZIEREN VON STICKSTOFFMONOXID IN DAS ATEMGAS EINES PATIENTEN**

(30) Priorität: 21.11.2024 DE 102024134222
(71) Anmelder: EKU Elektronik GmbH, 56291 Leiningen (DE)
(72) Erfinder: Köbrich, Rainer, 35321 Laubach (DE)
(74) Vertreter: Münzel, Joachim R.

(57) **Zusammenfassung**

Zum Applizieren eines Stickstoffmonoxid enthaltenden Applikationsgases in das Atemgas eines Patienten wird der Patient (7) über eine mit einem Beatmungsinterface (9) strömungsverbundenen Atemgaszuleitung (6) mit einem Atemgas versorgt und ein Stickstoffmonoxid enthaltendes Applikationsgas aus einer Gasquelle (2) über eine in die Atemgaszuleitung und/oder das Beatmungsinterface einmündende Applikationsgaszuleitung (3) in das Atemgas eingeleitet. Erfindungsgemäß wird der der Gasstrom des Applikationsgases mittels der Steuereinrichtung (12) derart geregelt, dass die Zuführung von Applikationsgas für eine vorgegebene Zeitdauer unterbrochen und anschließend wiederaufgenommen wird, um den Rebound des Patienten in Bezug auf die artifizielle iNO-Gabe testen zu können

## Beschreibung

Die Erfindung betrifft eine Vorrichtung zum Applizieren von Stickstoffmonoxid in das Atemgas eines Patienten, mit einer Einrichtung zum Zuführen eines Atemgases an einen Patienten, die mit einer Atemgaszuleitung und einem Beatmungsinterface ausgerüstet ist, mit einer Einrichtung zum Einleiten eines Stickstoffmonoxid enthaltenden Applikationsgases in das Atemgas, die mit einer Gasquelle für das Applikationsgas und mit einer in die Atemgaszuleitung und/oder in das Beatmungsinterface einmündenden Applikationsgaszuleitung ausgerüstet ist, und mit einer Steuereinrichtung zur Regulierung des durch die Applikationsgaszuleitung strömenden Gasflusses, die mit einem Steuerventil in der Applikationsgaszuleitung zusammenwirkt.

Vorrichtungen zum Applizieren von Stickstoffmonoxid (NO) oder Stickstoffmonoxid enthaltenden Gasen sind bekannt.

So beschreibt die US 2005 076 907 A1 ein Verfahren und eine Vorrichtung zum Applizieren von Stickstoffmonoxid an einen Patienten, bei dem das Strömungsprofil des zugeführten NO-haltigen Gases während der Inspiration des Patienten proportional zum Strömungsprofil des Atemgases eingestellt wird. Dadurch wird eine gleichbleibende Konzentration an Stickstoffmonoxid im zugeführten Atemgas zu jedem Zeitpunkt der Atemtätigkeit gewährleistet.

Die EP 3 097 938 A1 betrifft eine Vorrichtung zur Applikation eines medizinischen Gases, insbesondere von Stickstoffmonoxid. Bei dieser Vorrichtung wird die Zugabe des medizinischen Gases in Abhängigkeit von einem Atemprofil eines Patienten gesteuert. Dabei wird die Menge und der Zeitpunkt der Zuführung des medizinischen Gases so bestimmt, dass die Gabe des medizinischen Gases individuell an die Atemtätigkeit des Patienten und die gewünschte Zielstelle der Einwirkung des medizinischen Gases in der Lunge angepasst werden kann.

In der WO 95/10315 wird vorgeschlagen, Stickstoffmonoxid einem Patienten derart geregelt zuzuführen, dass sich im Körper des Patienten kein Stickstoffdioxid bilden kann. Dazu wird das Stickstoffmonoxid dem Patienten intermittierend und in sehr kurzen Pulsen zu Beginn oder gegen Ende jeder Einatmung verabreicht.

Aus der DE 10 2010 016 699 A1 ist eine Vorrichtung zur Applikation eines medizinischen Gases, insbesondere eines Stickstoffmonoxid enthaltenden Gases, bekannt, bei der das medizinische Gas in Form rasch aufeinanderfolgender Pulse in das Atemgas eines Patienten eingespeist wird. Die Taktfrequenz der Pulse ist dabei höher als die Atemfrequenz des Patienten. Die durch die kurzen Gaspulse bewirkte Gaseinmischung führt im Körper des Patienten zu einem weitgehend homogenen Partialdruck des Stickstoffmonoxids im Atemgas. Durch Variation der Pulslänge und Taktfrequenz können insbesondere kleine Dosierungen sehr genau eingestellt werden.

Bei einer länger andauernden inhalativen Stickstoffmonoxid-Therapie (iNO-Therapie) stellt sich bei intensivmedizinisch therapierten Patienten im Verlauf der Behandlung eine Medikamentenabhängigkeit ein, die bei einer plötzlichen Unterbrechung der kontinuierlichen Therapiegaszuführung zur klinischen Verschlechterung bis hin zur lebensbedrohlichen Krise führen kann. Dieser sogenannte Rebound-Effekt entsteht dadurch, dass die Bereitstellung von artifiziellen Stickstoffmonoxid in das Atemgas die körpereigene Stickstoffmonoxid-Produktion unterdrückt. Wird, beispielsweise bei Beendigung der Therapie, die Versorgung mit Stickstoffmonoxid abrupt eingestellt, gelingt es dem Körper nicht rasch genug, das fehlende Behandlungsgas zu ersetzen. Aufgrund der dann fehlenden vasodilatatorischen Wirkung des Stickstoffmonoxids kommt zu einer akuten Sauerstoffunterversorgung des Patienten durch Erhöhung des Strömungswiderstands im Lungenkreislauf, mit der Folge einer verstärkten Rechts-Herz-Belastung. Um diese klinische Krise zu vermeiden, wird in der üblichen Routine der Patient einer Entwöhnungsprozedur unterworfen, bei der die Stickstoffmonoxid-Konzentration im Atemgas stufenweise reduziert wird.

Mit den zurzeit verfügbaren Therapiegeräten gibt es kein quantifizierbares Signal, wann die Medikamentierung beendet werden kann, ohne in einen Rebound zu geraten. Stattdessen entscheidet der behandelnde Arzt aufgrund von persönlichen Erfahrungswerten, wann und wie die Zugabe von Stickstoffmonoxid reduziert und schließlich eingestellt wird. Im Zweifel führt dies zu einer eigentlich unnötigen Verlängerung der Therapie und/oder zu einem Mehrverbrauch an therapeutischem Gas.

Der Erfindung liegt daher die Aufgabe zu Grunde, eine Vorrichtung zur Applikation eines Stickstoffmonoxid enthaltenden Gases in das Atemgas eines Patienten im Rahmen einer iNO-Therapie zu entwickeln, die eine verbesserte Einschätzung eines zu erwartenden Rebound-Effekts und eine effizientere Dosierung des Applikationsgases ermöglicht.

Gelöst ist diese Aufgabe durch eine Vorrichtung mit den Merkmalen des Patentanspruchs 1. Vorteilhafte Ausgestaltungen der Erfindung sind in den Unteransprüchen angegeben.

Eine Vorrichtung der eingangs genannten Art und Zweckbestimmung ist erfindungsgemäß dadurch gekennzeichnet, dass das Steuerventil durch ein in der Steuereinrichtung vorliegendes Steuerprogramm derart ansteuerbar ist, dass in einem Normalbetriebszustand das Steuerventil geöffnet ist und ein einer vorgegebenen Dosierung entsprechender Gasstrom durch die Applikationsgaszuleitung geführt wird, jedoch während wenigstens eines Unterbrechungsintervalls für eine vorgegebene Intervalldauer von mindestens 15s das Steuerventil gesperrt ist und keine Zuführung von Applikationsgas über die Applikationszuleitung erfolgt.

Im Verlauf einer iNO-Therapie wird also ein Patient über die mit einem Beatmungsinterface (etwa einer Atemmaske oder einen Beatmungstubus) strömungsverbundene Atemgaszuleitung mit einem Atemgas versorgt, und ein Stickstoffmonoxid enthaltendes Applikationsgas aus einer Gasquelle wird über die in die Atemgaszuleitung oder das Beatmungsinterface einmündende Applikationsgaszuleitung kontinuierlich oder in Form kurzer Gaspulse in das Atemgas des Patienten eingespeist. Die Einleitung des Applikationsgases in das Atemgas erfolgt nach einem Steuerprogramm, das in der elektronischen Steuereinrichtung (oder in einem mit der Steuereinrichtung drahtgebunden oder drahtlos kommunizierenden Überwachungssystem) vorliegt. Beispielsweise liegt das Steuerprogramm in Gestalt eines Computerprogramms vor, das in einer zentralen Rechnereinheit der Steuereinheit einprogrammiert ist. Im Normalbetriebszustand ermöglicht das Steuerprogramm so die Einleitung des Applikationsgases entsprechend einer therapeutisch vorgesehenen Dosierung; die jeweilige Dosierung richtet sich insbesondere nach der therapeutischen Einstellung des behandelnden Arztes.

Erfindungsgemäß erfolgt im Verlauf der Therapie mindestens eine zeitlich beschränkte Unterbrechung (hier auch "Unterbrechungsintervall" genannt) der Zuführung des Applikationsgases für eine Dauer (hier auch "Intervalldauer" genannt) von mindestens 15s. Nach Ablauf eines Unterbrechungsintervalls wird die Therapie in der zuvor eingestellten Dosierung automatisch fortgesetzt.

Zu Beginn und zum Ende des Unterbrechungsintervalls wird vom Steuerprogramm ein Steuerbefehl zum Schließen bzw. Öffnen des Steuerventils in der Applikationsgaszuleitung veranlasst, sodass während der Intervalldauer kein Applikationsgas in das Atemgas eingeleitet wird. Die Intervalldauer kann vor Beginn der Therapie im Steuerprogramm voreingestellt werden und/oder sie ist bereits als Standardwert für eine gegebene Fallsituation im Steuerprogramm enthalten, die dazu ausgewählt werden muss. Eine etwaig im Steuerprogramm vorgesehene Alarmierung, die im Falle einer plötzlichen Veränderung des zugeführten Mengenstroms an Applikationsgas ein Notsignal absendet, wird für die Dauer des Unterbrechungsintervalls inaktiviert. Das Steuerprogramm kann Teil eines Applikationsprogramms sein, in welchem zugleich ein zeitlicher Ablauf einer Dosierung des Applikationsgases im Normalbetriebszustand festgelegt ist.

Der Beginn des Unterbrechungsintervalls und die Intervalldauer sind unabhängig von der Atemtätigkeit des Patienten. Die minimale Intervalldauer von 15 s bemisst sich an der Lebensdauer von NO-Molekülen, deren Halbwertszeit in biologischen Systemen typischerweise nur wenige Sekunden beträgt. Nach einer Zeitdauer von 15s kann davon ausgegangen werden, dass im Atemgas des Patienten NO-Moleküle aus dem zuvor zugeführten Applikationsgas nicht mehr oder nur in vernachlässigbar geringer Menge enthalten sind. Stickstoffmonoxid kann also nur noch über körpereigene Prozesse gebildet werden. Während des Unterbrechungsintervalls werden die Körperfunktionen des Patienten kontinuierlich überwacht und ermöglichen es so, die Medikamentenabhängigkeit des Patienten zu ermitteln und über den weiteren Verlauf der Therapie zu entscheiden. Durch die Wiederaufnahme der Zuführung von Applikationsgas im Anschluss an das Unterbrechungsintervall wird zugleich sichergestellt, dass beim Patienten eine Krise der vorgenannten Art verhindert wird.

Bevorzugt ist das Steuerprogramm so eingerichtet, dass eine Intervalldauer jeweils zwischen 30 s und 600 s beträgt. Eine Zeitdauer von 30s stellt sicher, dass kein die Produktion von körpereigenem Stickstoffmonoxid störendes Applikationsgas mehr in der Lunge des Patienten vorhanden ist. Eine Intervalldauer von über 600s kann bei Patienten, die auf die Zufuhr von artifiziellem Stickstoffmonoxid angewiesen sind, zu einer zu starken Belastung der Lungenfunktion führen.

Bevorzugt ist die Steuereinrichtung mit wenigstens einer Messeinrichtung zur Erfassung einer NO-Konzentration im Atemgas und/oder zur Erfassung von Körperfunktionen des Patienten datenverbunden. Die Messdaten werden der Steuereinrichtung zur Verarbeitung zugeführt und können beispielsweise auf einem mit der Steuereinrichtung datenverbundenen Bildschirm visualisiert werden. Bevorzugt ist dabei das Steuerprogramm derart eingerichtet, dass aus den erfassten Messdaten therapierelevante Informationen gewonnen werden, wie beispielsweise Informationen über einen Rebound beim Patienten, eine für die weitere Therapie geeignete Dosierung des Applikationsgases und/oder eine Intervalldauer des Unterbrechungsintervalls. Wird beispielsweise festgestellt, dass während des Unterbrechungsintervalls beim Patienten eine verstärkte Eigenerzeugung von Stickstoffmonoxid eingesetzt hat, kann das Unterbrechungsintervall verlängert werden, wird dagegen festgestellt, dass die Unterbrechung der Zuführung die Applikationsgases zu einer rapiden Verschlechterung des Zustandes des Patienten führt, wird das Unterbrechungsintervall abgekürzt und die Zuführung des Applikationsgases wieder aufgenommen.

Weiterhin kann die Steuereinrichtung mit einem Bildschirm in Datenverbindung stehen, auf dem die Restdauer eines laufenden Unterbrechungsintervalls angezeigt werden kann, ggf. zusätzlich zu einer Visualisierung gemessener Parameter. Die Steuereinrichtung kann darüber hinaus mit einem Bedienpaneel datenverbunden sein, das dem Therapeuten die Eingabe von Steuerinformationen, wie beispielsweise eine Auswahl von Steuerprogrammen und/oder einen Eingriff in das Steuerprogramm ermöglicht. Zu diesem Zweck kann ein mit dem Steuerprogramm datenverbundener Bildschirm auch als Touchscreen ausgebildet sein.

Eine besonders vorteilhafte Ausgestaltung der Erfindung sieht vor, dass die Steuereinrichtung eine Notschaltung aufweist, die im Falle eines vorbestimmbaren Ereignisses automatisch und/oder durch manuellen Eingriff durch den Therapeuten das Steuerprogramm der Steuereinrichtung übersteuert und eine Zufuhr von Stickstoffmonoxid während eines Unterbrechungsintervalls ermöglicht. Bevorzugt ist die Notschaltung zudem mit einer Einrichtung zur akustischen und/oder optischen Alarmierung wirkverbunden. Als "vorbestimmbares Ereignis" kommt insbesondere das Über- oder Unterschreiten eines bestimmten Parameters in Betracht, etwa der Wert einer Körperfunktion des Patienten, wie beispielsweise Blutdruck oder Herzfrequenz, der an einer mit der Steuereinrichtung datenverbundenen Messeinrichtung erfasst wurde.

Die erfindungsgemäße Vorrichtung eignet sich daher besonders für einem effizienten Abschluss einer iNO-Therapie. Dazu werden ggf. mehrere Unterbrechungsintervalle in zeitlichen Abständen hintereinander durchgeführt und jeweils geprüft, in welchem Umfang weiterhin eine Medikamentenabhängigkeit besteht und/oder wie weit die Entwöhnung (Weaning) des Patienten fortgeschritten ist. Auch hier kann die Überprüfung automatisiert erfolgen, indem beispielsweise während eines jeden Unterbrechungsintervalls die NO-Konzentration im Atemgas und/oder bestimmte Körperfunktionen kontinuierlich gemessen und im weiteren Verlauf der Therapie als Richtgröße für ein mögliches Absenken der Dosierung des Applikationsgases und/oder für einer möglichen Verlängerung der Unterbrechungsintervalle eingesetzt werden kann/können.

Bevorzugt liegt in der Steuereinrichtung eine Mehrzahl von Steuerprogrammen vor, die unterschiedlichen Fallsituationen angepasst sind, bei denen jeweils der jeweilige Patiententyp (beispielsweise Neugeborenes, Heranwachsender, Erwachsener) sowie die jeweilige Indikation (beispielsweise Persistierende pulmonale Hypertonie beim Neugeborenen oder Pulmonale Hypertonie im Zusammenhang mit einer Herzoperation) berücksichtigt wird. Das jeweils zum Einsatz kommende Steuerprogramm wird vor Beginn der Therapie aufgerufen. Dabei kann vorgesehen sein, dass das eingespeicherte Steuerprogramm durch einen Therapeuten manuell an individuelle Erfordernisse des Patienten angepasst werden kann.

Zweckmäßigerweise ist das Steuerprogramm so eingerichtet, dass das Applikationsgas außerhalb des Unterbrechungsintervalls, also im Normalbetriebszustand, kontinuierlich zugeführt wird. Alternativ zur kontinuierlichen Zuführung kann das das Applikationsgas auch in Form von rasch aufeinanderfolgenden Gaspulsen zugeführt werden, die zu einem zeitlich weitgehend homogenen Partialdruck des Applikationsgases im zugeführten Atemgas führen. Wesentlich für die Erfindung ist jedoch, dass während des Unterbrechungsintervalls kein Applikationsgas, auch nicht in Form von Gaspulsen, zugeführt wird.

Die erfindungsgemäße Vorrichtung kommt beispielsweise in der Neonatologie zur iNO-Behandlung einer pulmonalen Hypertonie bei Neugeborenen, zur Behandlung von Kindern und Erwachsenen bei einer pulmonalen Hypertonie im Zusammenhang mit einer Herzoperation oder zur Behandlung von Patienten nach einer Organtransplantation zum Einsatz. Als bevorzugtes Applikationsgas kommt Stickstoffmonoxid oder ein Stickstoffmonoxid enthaltendes Gasgemisch zum Einsatz.

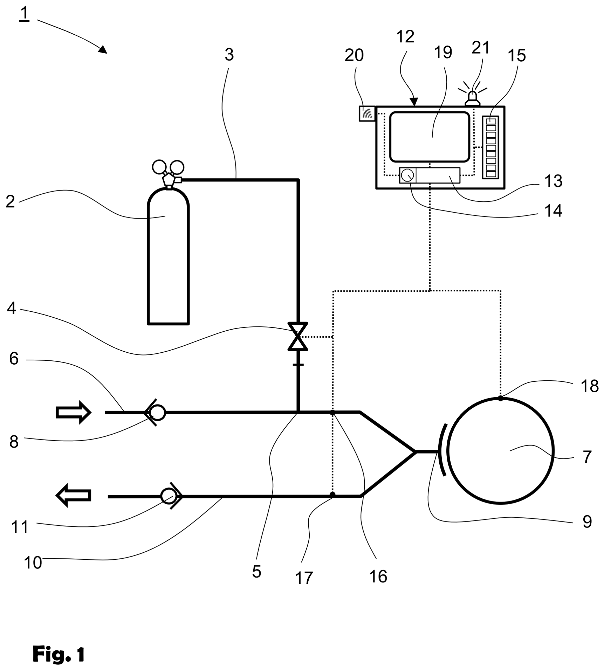

Anhand der Zeichnung soll ein Ausführungsbeispiel der Erfindung näher erläutert werden. Die einzige Zeichnung (Fig. 1) zeigt eine schematische Darstellung einer erfindungsgemäßen Vorrichtung zur Applikation eines Stickstoffmonoxid enthaltenden Applikationsgases an einen Patienten.

Die in Fig. 1 gezeigte Vorrichtung 1 weist eine Quelle, insbesondere eine Druckgasflasche 2, zur Bereitstellung eines Stickstoffmonoxid enthaltenden Applikationsgases auf. Beim Applikationsgas handelt es sich beispielsweise um reines Stickstoffmonoxid oder um ein Gasgemisch, beispielsweise bestehend aus Stickstoffmonoxid sowie Stickstoff und/oder einem Edelgas. Von der Druckgasflasche 2 führt eine Applikationsgaszuleitung 3 ab, die mit einem Steuerventil 4 ausgerüstet ist. Stromab zum Steuerventil 4 mündet die Applikationsgaszuleitung 3 an einer Mischstelle 5 in eine Atemgaszuleitung 6 ein.

Die Atemgaszuleitung 6 dient zur Zuführung eines Atemgases von einem hier nicht gezeigten Beatmungsgerät an einen Patienten 7. Dabei verhindert ein Rückschlagventil 8 in der Atemgaszuleitung 6, dass ausgeatmetes Gas vom Patienten 7 durch die Atemgaszuleitung 6 zurückströmt. Beim Atemgas handelt es sich beispielswiese um ein Luft-Sauerstoffgemisch, medizinische Luft (*aer medicalis*) oder um ein Sauerstoff-Distickstoff-Gemisch. Die Atemgaszuleitung 6 mündet in ein dem Patienten 7 aufgesetztes Beatmungsinterface 9, beispielsweise eine Atemmaske, ein. Von dem Beatmungsinterface 9 führt zudem eine Atemgasausleitung 10 zum Abführen des vom Patienten 6 ausgeatmeten Atemgases ab, in welcher ein druckregulierendes Ventil 11 verhindert, dass während der Atemtätigkeit des Patienten 7 ausgeatmete Luft zum Patienten 7 zurückströmt.

Die Vorrichtung 1 weist des Weiteren eine elektronische Steuereinrichtung 12 auf, mittels der in der im Folgenden näher beschriebenen Weise die Zufuhr des Applikationsgases an den Patienten 7 gesteuert werden kann. Im Übrigen können verschiedene Teile der Vorrichtung 1, insbesondere die Druckgasflasche 2, die elektronische Steuereinrichtung 12 und zumindest Abschnitte der Applikationsgaszuleitung 3 und mit der Steuereinrichtung 12 verbundene elektronische Leitungen in einem gemeinsamen Gehäuse oder in einem gemeinsamen Gestell angeordnet sein, was indes hier nicht gezeigt ist.

Im Betrieb der Vorrichtung 1 wird dem Patienten 7 über die Atemgaszuleitung 6 ein Atemgas zugeführt. Gleichzeitig wird über die Applikationsgaszuleitung 3 ein Stickstoffmonoxid enthaltendes Applikationsgas in die Atemgaszuleitung 6 kontinuierlich oder in Form kurzer Gaspulse eingespeist (Normalbetriebszustand). In der Atemgaszuleitung 6 vermischt sich das Applikationsgas mit dem Atemgas derart, dass dem Patienten über das Beatmungsinterface 9 ein Atemgasgemisch mit einem weitgehend gleichbleibenden Partialdruck an Applikationsgas zugeführt wird. Das ausgeatmete Atemgas des Patienten 7 wird über die Atemgasausleitung 10 abgeführt.

Die Steuereinrichtung 12 ist mit einer elektronischen Rechnereinheit 13 ausgerüstet, in der ein Steuerprogramm in Form eines Computerprogramms vorliegt. Das Steuerprogramm regelt den Mengenstrom des über die Applikationszuleitung 6 zugeführten Applikationsgases im Normalbetriebszustand durch entsprechende Steuerbefehle an das Steuerventil 4, entsprechend einer im Algorithmus des Steuerprogramms vorgegebenen Dosierung, die sich insbesondere an der jeweiligen Indikation und/oder dem Patiententyp orientiert.

Weiterhin ist die Rechnereinheit 13 mit einem Zeitgeber 14 ausgerüstet, und das Steuerprogramm ist so ausgebildet, dass die Zufuhr des Applikationsgases während eines Unterbrechungsintervalls für eine vorgegebene Zeitdauer (Intervalldauer) unterbrochen wird, während der kein Applikationsgas zugeführt wird. Zu Beginn und zum Ende des Unterbrechungsintervalls wird dazu von der Steuereinrichtung 12 ein Steuerbefehl abgegeben, mittels dessen das Steuerventil 4 geschlossen bzw. geöffnet wird. Die Intervalldauer sollte so bemessen sein, dass sie eine Mehrzahl von Atemzügen eines behandelten Patienten überspannt, zumindest aber ist sie größer als 15s, um sicherzustellen, dass im Atemgas des Patienten 7 kein Applikationsgas mehr vorliegt; bevorzugt wird die Einleitung des Applikationsgases für eine Dauer von 30s bis 600s unterbrochen. Nach Beendigung des Unterbrechungsintervalls gelangt die Vorrichtung 1 automatisch in den Normalbetriebszustand zurück, und Applikationsgas wird entsprechend der vorgegebenen Dosierung über die Applikationsgaszuleitung 3 zugeführt.

Die Intervalldauer wird im Steuerprogramm unter Berücksichtigung der jeweiligen Situation voreingestellt. Dies kann manuell an einem mit der Rechnereinheit 13 datenverbundenen Bedienpanel 15 erfolgen, oder in der Rechnereinheit 13 ist bereits ein Steuerprogramm eingespeichert, in welchem Standardwerte für bestimmte Therapien und/oder bestimmte Patiententypen vorgegeben sind.

Zumindest während des Unterbrechungsintervalls werden die Körperfunktionen des Patienten 7 kontinuierlich überwacht und geprüft, ob und in welchem Maß beim Patienten weiterhin eine Abhängigkeit von der Zuführung des Stickstoffmonoxid enthaltenden Applikationsgases besteht. Dazu ist die Steuereinrichtung 12 mit einer oder mehreren Messeinrichtungen zur Erfassung bestimmter, für die Therapie wesentlicher Parameter datenverbunden; im hier gezeigten Ausführungsbeispiel werden an einem ersten Messpunkt 16 die Zusammensetzung des dem Patienten 7 zugeführten Atemgases in der Atemgaszuleitung 6 stromab zur Mischstelle 5 und an einem zweiten Messpunkt 17 die Zusammensetzung des vom Patienten 7 ausgeatmeten Atemgases in der Atemgasausleitung 10 erfasst. An einem weiteren Messpunkt 18 werden Daten des Patienten 7 erfasst, wie beispielsweise Blutdruck, Atem- und/oder Herzfrequenz. Selbstverständlich können im Rahmen der Erfindung auch keine oder nur einzelne dieser Messdaten oder auch zusätzliche Messdaten erfasst werden. Die Messdaten und/oder weitere Patientendaten und/oder die verbleibende Zeitdauer eines laufenden Unterbrechungsintervalls können an einem Bildschirm 19 visualisiert werden, der mit der Rechnereinheit 13 in Datenverbindung steht. Der Bildschirm 19 kann im Übrigen auch als Touchscreen ausgebildet sein, in den das Bedienpaneel 15 integriert ist. Weiterhin können vom Steuerprogramm aus den gemessenen Daten Sollwerte für eine gegebenenfalls veränderte Dosierung und/oder für eine verlängerte oder verkürzte Intervalldauer errechnet werden, die die Grundlage für die weitere Therapie bilden.

Optional kann die Steuereinrichtung 12 mit einer Kommunikationseinrichtung 20 zur Fernsteuerung und/oder zur Übermittlung von Daten aus der Rechnereinheit 13 an eine externe (hier nicht gezeigte) Steuereinheit ausgerüstet sein. Ebenso kann ein mit der Rechnereinheit 13 in Datenverbindung stehender Notsignalgeber 21 vorhanden sein, der bei Eintreten eines vorherbestimmten Ereignisses, etwa im Falle einer plötzlichen Verschlechterung des Gesundheitszustands des Patienten 7, ein akustisches und/oder optisches Notsignal abgibt. Für einen solchen Fall ist das Steuerprogramm so eingerichtet, dass es bei Eintreten eines derartigen Ereignisses manuell oder von einem automatisch ablaufenden Notfallprogramm übersteuert werden kann.

### Bezugszeichenliste

- 1: Vorrichtung
- 2: Druckgasflasche
- 3: Applikationsgaszuleitung
- 4: Steuerventil
- 5: Mischstelle
- 6: Atemgaszuleitung
- 7: Patient
- 8: Rückschlagventil
- 9: Beatmungsinterface
- 10: Atemgasausleitung
- 11: Ventil
- 12: Steuereinrichtung
- 13: Rechnereinheit
- 14: Zeitgeber
- 15: Bedienpanel
- 16: Messpunkt
- 17: Messpunkt
- 18: Messpunkt
- 19: Bildschirm
- 20: Kommunikationseinrichtung
- 21: Notsignalgeber

## Patentansprüche

1. Vorrichtung zum Applizieren von Stickstoffmonoxid in das Atemgas eines Patienten, mit einer Einrichtung zum Zuführen eines Atemgases an einen Patienten (7), die mit einer Atemgaszuleitung (6) und einem Beatmungsinterface (9) ausgerüstet ist, mit einer Einrichtung zum Einleiten eines Stickstoffmonoxid enthaltenden Applikationsgases in das Atemgas, die mit einer Gasquelle (2) für das Applikationsgas und mit einer in die Atemgaszuleitung (6) und/oder in das Beatmungsinterface (9) einmündenden Applikationsgaszuleitung (3) ausgerüstet ist, und mit einer Steuereinrichtung (12) zur Regulierung des durch die Applikationsgaszuleitung (3) strömenden Gasflusses, die mit einem Steuerventil (4) in der Applikationsgaszuleitung (3) zusammenwirkt,
**dadurch gekennzeichnet,**
**dass** das Steuerventil (4) durch ein in der Steuereinrichtung (12) vorliegendes Steuerprogramm derart ansteuerbar ist, dass in einem Normalbetriebszustand das Steuerventil (4) geöffnet ist und ein einer vorgegebenen Dosierung entsprechender Gasstrom durch die Applikationsgaszuleitung (3) geführt wird, jedoch während wenigstens eines Unterbrechungsintervalls für eine vorgegebene Intervalldauer von mindestens 15s das Steuerventil (4) gesperrt wird und keine Zuführung von Applikationsgas über die Applikationsgaszuleitung (3) erfolgt.

2. Vorrichtung nach Anspruch 1, **dadurch gekennzeichnet, dass** das Steuerprogramm so eingerichtet ist, dass eine Intervalldauer eines Unterbrechungsintervalls zwischen 30s und 600s beträgt.

3. Vorrichtung nach einem der vorhergehenden Ansprüche, **dadurch gekennzeichnet, dass** die Steuereinrichtung (12) mit Messeinrichtungen (16, 17, 18) zur Erfassung einer Stickstoffmonoxid-Konzentration im Atemgas und/oder zur Erfassung von Körperfunktionen des Patienten datenverbunden ist.

4. Vorrichtung nach einem der vorhergehenden Ansprüche, **dadurch gekennzeichnet, dass** die Steuereinrichtung (12) mit einem Bildschirm (19) und/oder mit einem Bedienpaneel (15) zur Eingabe von Steuerinformationen in Datenverbindung steht.

5. Vorrichtung nach einem der vorhergehenden Ansprüche, **dadurch gekennzeichnet, dass** die Steuereinrichtung (12) eine Notschaltung umfasst, die im Falle eines vorbestimmten Ereignisses das Steuerprogramm übersteuert und die Öffnung des Steuerventils (4) in der Applikationszuleitung (3) durch manuellen Eingriff und/oder durch Starten eines Notfallprogramms erlaubt.

6. Vorrichtung nach einem der vorhergehenden Ansprüche, **dadurch gekennzeichnet, dass** in der Steuereinrichtung (12) eine Mehrzahl an Steuerprogrammen vorliegt, in denen jeweils für bestimmte Therapiefälle typische Werte für Intervalldauern voreingestellt sind.

7. Vorrichtung nach einem der vorhergehenden Ansprüche, **dadurch gekennzeichnet, dass** das Steuerprogramm so eingerichtet ist, dass das Applikationsgas im Normalbetriebszustand kontinuierlich oder in Form von Gaspulsen zugeführt wird, deren Anzahl pro Zeiteinheit höher als die Atemfrequenz des Patienten (7) ist.

8. Vorrichtung nach einem der vorhergehenden Ansprüche, **dadurch gekennzeichnet, dass** als Applikationsgas Stickstoffmonoxid oder ein Stickstoffmonoxid enthaltendes Gasgemisch vorgesehen ist.
